# EUROPEAN PATENT APPLICATION

(11) **EP 1 623 988 A1**
(43) Date of publication of application: **08.02.2006**
(21) Application number: 04732485.0
(22) Date of filing: 12.05.2004
(51) Int. Cl.: C07K 1/13

(54) **PROTEIN REFOLDING APPARATUS AND METHOD OF USING THE SAME**

(30) Priority: 13.05.2003 JP 2003135108; 19.05.2003 US 471549 P
(71) Applicant: Universal Bio Research Co., Ltd., Matsudo-shi, Chiba 271-0064 (JP); Proteomtech, Inc., Emeryville, CA 94608 (US)
(72) Inventor: TAJIMA, Hideji c/o Universal Bio Research Co.,Ltd, Matsudo-shi, Chiba 271-0064 (JP); LIN, Xinli c/o ProteomTech, Inc., Suite 405, Emeryville, CA 94608 (US); BURROWES, Peter c/o Bio-Strand, Inc., Suite H, Pleasanton, CA 94566 (US); KUSUMOTO, Chris c/o PSS Bio Instruments, Inc., Suite H, Pleasanton, CA 94566 (US)
(74) Representative: Bohnenberger, Johannes
(86) International application number: PCT/JP2004/006681
(87) International publication number: WO 2004/101601

(57) **Abstract**

An apparatus for refolding proteins and a method of using an apparatus for refolding proteins is provided in which a refolding process for proteins can be carried out for various proteins, efficiently and accurately, and without manual intervention. The construction comprises: a transport section on which either one or two or more vessels capable of holding a protein solution containing a protein to be refolded are placed, which transports the vessels along a predetermined closed transport path; an optical measurement section provided on the transport path, which measures the optical properties of the protein solution within the vessel; a pH measuring section provided on the transport path, which measures the pH of the protein solution within the vessel; and a reagent supply section provided on the transport path, capable of supplying each of a plurality of types of reagent including reagents for adjusting the pH of the protein solution within the vessel, wherein the reagent supply section performs protein refolding by adjusting the pH of the protein solution based on the measurement results of the optical measurement section, the measurement results of the pH measuring section, and a predetermined pH pattern.

## Description

### TECHNICAL FIELD

The present invention relates to an apparatus for refolding proteins and a method of using an apparatus for refolding proteins. The present invention is used in the field of methods for the manufacture of recombinant proteins, and especially in the field of refolding (unfolding the incorrect structures of inactive protein aggregates which have lost their proper activity and refolding them into the correct three dimensional structure) of recombinant proteins expressed in the inclusion bodies of prokaryotic expression systems such as E. coli (colon bacillus).

Expression of proteins with natural biological activity and structure, referred to as "proteomics", has become increasingly important with the completion of genomic sequencing for several organisms and the human genome. One aspect of proteomics is to express large amounts of protein for structural and functional studies, as well as for commercial applications. An inexpensive and efficient way to express recombinant proteins is to express the proteins in E. coli. Proteins are expressed either intracellularly or secreted into the periplasmic space. In the case of the former, the proteins are often deposited in inclusion bodies, especially if the protein has disulfide bonds.

However, one of the problems in expressing mammalian proteins in E. coli is that most of the expressed proteins form insoluble inclusion bodies. While this problem can be circumvented by using various mammalian or insect expression systems, growing E. coli is faster and less expensive compared to mammalian and insect cultures. Moreover, some proteins are toxic to the host when expressed in their native forms, and thus expression as insoluble inclusion bodies is the only way to obtain large quantities of recombinant proteins. Importantly, high levels of expression can be achieved for most proteins. 400 to 600 mg of inclusion bodies per liter of bacterial culture can routinely be achieved, with up to 9,700 mg/L having been reported using this method (Jeong KL; Lee SY, 1999. Appl. Environ. Macrobiol. 65:3027-32). Inclusion bodies can be easily purified to greater than 90% with a simple freeze/thaw and surface active detergent washing procedure.

Inclusion bodies appear as dense Bombyx mori cytoplasmic granules when the cells are observed under a light microscope. Typically, the cells will be lysed by mechanical disruption of the cells, followed by centrifugation for 30 min at 4700 g. Inclusion bodies will sediment at low g forces and can be separated from many other intracellular proteins. In addition, further purification can be achieved by washing the pellet with the buffer used during the cell disruption, or by centrifuging the resuspended pellet in 40-50% glycerol.

Many extracellular proteins of eukaryotes contain disulfide bonds. Proteins having multiple disulfide bonds may form non-native disulfide bonds during folding from the formed structure. Further folding is then blocked unless the incorrect disulfide bond is cleaved by reduction with an external thiol or by attack from a protein thiol. A distinct disadvantage of expression of recombinant proteins in prokaryotes as inclusion bodies is that the proteins are not obtained in their native state, and typically are not functionally active.

### BACKGROUND ART

Conventionally, a variety of methods have been used to lyse the proteins and refold them to reform active protein. Dissolution of the pelleted recombinant protein usually requires the use of denaturants such as 7 M (molar concentration) guanidine hydrochloride or 8 M urea. The amount of aggregation may continue to increase if the protein is allowed to remain in the denaturant (Kelley and Winkler, "Folding of Eukaryotic Proteins Produced in Escherichia coli" Genetic Engineering 12, 1-19 at p. 6 (1990), indicated by "Document 1" below). Removal of the denaturant from the solubilized inclusion bodies by dialysis or desalting Zcolumns will cause the protein to precipitate under the conditions required for the native protein to be refolded. A misfolded protein solution can also have very low specific activity in biological assays.

Although there are many reports of expression and refolding of various proteins in E. coli as inclusion bodies, one of the misconceptions in protein refolding is that a unique refolding method has to be developed for each individual protein (Document 1). Another misconception is that the majority of mammalian proteins cannot be refolded from inclusion bodies (Rudolph R., Lilie H., 1996. FASEBJ 10:49-56, indicated by "Document 2" below, Lilie H, Schwarz E, Rudolph R. 1998. Curr. Opin. Biotechnol 9;497-501, indicated by "Document 3" below). Because published works are mostly "success" stories in refolding inclusion bodies from E. coli, it is impossible to get a general idea about what percentage of mammalian proteins can be purified using this procedure.

There are many more refolding methods than refolded proteins reported in the literature (Document 2 and Document 3). Different chaperones, detergents and chaotrophs have been used to help refolding. In addition, pH, ionic strength, temperature, buffer formulation, and reducing/oxidizing reagents can all effect refolding. It would be prohibitive to test all these conditions for refolding large amounts of proteins, as required for studies in proteomics or structural genomics.

A single simplified procedure to refold most of the proteins that are expressed in recombinant systems, especially those which form inclusion bodies in systems such as E. coli, is therefore needed. As such a procedure, the present inventors have provided a method for refolding proteins, especially recombinant proteins, and especially recombinant proteins present in a bacterial host (International Publication Number WO 01/55174A2).

This method has two key steps, namely to unfold the proteins to be folded which are expressed as inclusion bodies, and to refold the proteins in biologically active form, with the correct three dimensional structure. The first step is to raise the pH of the protein solution in the presence of denaturing agents to a pH greater than 9, preferably 10. The protein solution may be maintained at the elevated pH for a period of up to approximately 24 hours, or the pH may be immediately decreased slowly, in increments of about 0.2 pH units/24 hours, until the solution reaches a pH of about 8.0, or both steps used. In the preferred embodiment, purified inclusion bodies are dissolved in 8 M urea, 0.1 M Tris (2-amino-2-hydroxymethyl-1, 3-propanediol has an extremely strong buffer action at around pH 8, and is used frequently as a buffer solution), 1 mM glycine, 1 mM EDTA (ethylenediaminetetraacetic acid), 10 mM beta-mercaptoethanol, 10 mM dithiothreitol (DTT), 1 mM reduced glutathione (GSH), 0.1 mH oxidized glutathione (GSSG), pH 10. The absorbance of light with wavelengths at 280 nm (OD₂₈₀) of the protein solution is 5.0. This solution is rapidly diluted to 20 times the volume of the 20 mM Tris base. The resulting solution is adjusted to a pH of 9.0 with 1 M HCl and kept at 4°C for 24 hours. The pH is adjusted to pH 8.8 and the solution is kept at 4°C for another 24 hours. This process is repeated until the pH is adjusted to 8.0. After 24 hours at pH 8.0, the refolded proteins can be concentrated by ultrafiltration and applied to a gel filtration column for purification.

However, as described above, in order to refold expressed proteins it is first necessary to unfold the expressed protein, and then refold the unfolded protein. Accordingly, it was necessary to repeat minute pH adjustments over along period of time.

Consequently, in order for this task to be carried out manually, an operator is required to monitor the conditions over a long period of time, and while measuring the pH of the solution, adjust the pH by adding at a suitable time, types and amounts of reagents containing a buffer solution determined in accordance with the measured pH, and this places a considerable burden on the operator.

Especially when performing a refolding process involving either a large amount or many types of proteins, the mechanical repetition of identical processes was necessary. Moreover the burden on the operator was increased dramatically because of the different conditions required when performing refolding processes for many types of proteins, these being particularly the different types of reagents, and the different amounts and different ratios thereof, and this causes a problem in that the processing cannot be carried out efficiently and quickly.

This leads to problems in that because these tasks are performed manually there is a danger of the processing not being highly reliable, and in that because the operator is occupied with the processing for a long period of time, higher personnel expenses lead to an increase in manufacturing costs.

Consequently, the present invention aims to resolve the problems outlined above. A first object of the present invention is to provide a refolding apparatus and a method of using the refolding apparatus, in which a refolding process for proteins, especially recombinant proteins, and especially recombinant proteins present in inclusion bodies in a bacterial host, can be carried out automatically and without manual intervention.

A second object is to provide a protein refolding apparatus and a method of using the protein refolding apparatus, in which the refolding of large amounts or many types of proteins can be performed efficiently, promptly and at low cost using a simple control.

A third object is to provide a highly reliable protein refolding apparatus and a method of using the protein refolding apparatus, in which the refolding process can be advanced by supplying reagents in an accurate and precise manner.

### DISCLOSURE OF THE INVENTION

In order to resolve the above problems, a first aspect of the invention is a protein refolding apparatus comprising: a transport section on which either one or two or more vessels capable of holding a protein solution containing a protein to be refolded are placed, which transports the vessels along a predetermined closed transport path; an optical measurement section provided on the transport path, which measures the optical properties of the protein solution within the vessel; a pH measuring section provided on the transport path, which measures the pH of the protein solution within the vessel; and a reagent supply section provided on the transport path, capable of supplying each of a plurality of types of reagent including reagents for adjusting the pH of the protein solution within the vessel, wherein the reagent supply section performs protein refolding by adjusting the pH of the protein solution based on the measurement results of the optical measurement section, the measurement results of the pH measuring section, and a predetermined pH pattern.

Here "optical properties" refers to a variety of optical properties, for example the properties of the transmitted light, reflected light or scattered light for different types of incident light (infrared light, far-infrared light, visible light and the like), or spectroscopic properties. An example of a property of transmitted light in relation to incident light is optical density (turbidity). Optical density is a value which expresses the degree to which the solute within the solution absorbs light, and is the logarithm of the reciprocal of the light absorption rate of the solute. In other words, it is expressed as log(I₀/I). Here, I₀ and I indicate the intensity of light passed through only the solvent, and the intensity of light passed through a solution layer with the same thickness, respectively. An increase in turbidity means an increase in the amount of aggregated protein which has unfolded and is suspended in the solution.

Accordingly, in the present invention, the refolding process is advanced by incorporating measurement results indicating the degree of protein unfolding, and adjusting the pH accordingly.

The "plurality of types of reagent for adjusting the pH of the protein solution" include at least buffer agents which maintain the pH at a constant level, acidifying reagents, and alkalinizing reagents. The pH is adjusted by using reagents belonging to these categories either individually or in combination as a mixture of suitable amounts of different reagents.

pH patterns are determined according to the protein being refolded. The general trend found in these patterns is that in a first stage the protein solution is maintained at a pH of greater that 9.0 in the presence of at least one chaotropic agent and reducing agent to unfold the aggregated protein, and in a second stage the pH of the solution is gradually reduced to 8.0 over a length of time which exceeds a predetermined length of time, and regeneration of at least part of the protein is induced until the protein shows biological activity and a characteristic structure, qualitatively, thereby refolding the unfolded protein to a three dimensional structure. The "predetermined length of time" depends on the temperature used in the processing. The temperature is set by a thermostatic device which is provided in the refolding apparatus and performs heating and cooling to maintain the transported vessels at a predetermined temperature. If for example the predetermined temperature is set to 4°C, the predetermined length of time becomes 24 hours, and if the temperature is set to 16°C, the predetermined length of time becomes 2 hours. According to the present invention, by changing the temperature of the thermostatic device, a variety of processing times, and accordingly a variety of pH patterns, can be obtained. In other words, the processing time can be lengthened or shortened. Furthermore, the reduction in the pH may involve reducing the pH in 0.2 increments each time the predetermined length of time passes, for example. Furthermore, the pH can be maintained above 9.0, as mentioned above, for the predetermined length of time or longer, for example.

The pH is reduced by adding an acid. The chaotropic reagent and denaturation reagent are selected from 0.5 and 1.0 M urea, 0.1 mM to 100 mM beta-mercaptoethanol, and 0.1 mM to 10 mM oxidized glutathione.

"Proteins" includes recombinant proteins, and also includes proteins extracted from inclusion bodies in bacterial hosts. These bacteria include E. coli. The inclusion bodies are dissolved at a pH between 9 and 10.

Because vessels are "transported along a closed transport path", the refolding process can be performed while monitoring the optical properties and pH for an individual vessel, and repeatedly adjusting the pH accordingly. The pH measuring section can be constructed comprising an electrode which can be inserted into and removed from the vessel, a cleaning section which cleans the electrode, and a moving section which enables movement of the electrode between the vessel and the cleaning section, for example.

According to the first aspect of the invention, the protein refolding process is performed non-manually, but without depending completely on a preset processing procedure, by measuring periodically the optical properties and pH value of the protein solution, and advancing the refolding process based on the measurement results. Accordingly, the refolding processes can be performed optimally for a variety of proteins.

According to the present invention, a refolding process can be performed efficiently, cheaply and promptly for large amounts or many types of proteins by simple control, without increasing the burden on the operator.

Furthermore, according to the present invention, by supplying accurate and precise amounts of reagents, calculated based on the measured optical properties and pH value, the refolding process can be advanced with high reliability.

A second aspect of the invention is a protein refolding apparatus, wherein the reagent supply section is provided downstream from the optical measurement section and the pH measuring section.

Preferably, the optical measurement section, the pH measuring section and the reagent supply section are provided adjacent to each other along the transport path in either the above order or in the order of the pH measuring section, the optical measurement section and the reagent supply section.

According to the second aspect of the invention, the reagent supply section is provided downstream from the optical measuring section and the pH measuring section in the transport path. Accordingly, the reagent supply section can supply the relevant reagents accurately and precisely based on the measurement results of the optical measurement section and the pH measuring section.

A third aspect of the invention is a protein refolding apparatus, wherein a stirring section which stirs the contents of the vessels is provided in the reagent supply section or in the transport path downstream from the reagent supply section.

According to the third aspect of the invention, the reagent supplied to the vessel is thereby mixed with the protein solution, enabling a reaction to be induced in an even and efficient manner.

A fourth aspect of the invention is a protein refolding apparatus wherein a suction and transport section which suctions a predetermined amount of a portion of the protein liquid contained in the vessel and transports it to the relevant vessel is provided in the transport path.

According to the fourth aspect of the invention, it is possible to be aware of the periodic progress of the refolding process, which is useful in the analysis and improvement of the refolding process.

A fifth aspect of the invention is a protein refolding apparatus, wherein the reagent supply section has at least three reagent discharge nozzles, provided so that at least any one of an acid reagent, an alkaline reagent and a buffer reagent can be discharged independently from each reagent discharge nozzle.

According to the fifth aspect of the invention, accurate pH adjustment is enabled by a simple construction, by the provision of at least three reagent discharge nozzles.

A sixth aspect of the invention is a protein refolding apparatus, wherein the stirring section stirs the liquid in the vessels on the transport path by rotating the vessel about the axis thereof

According to the sixth aspect of the invention, by stirring the liquid within a vessel by rotating the vessel, stirring can be accomplished by a simple construction and simple control.

A seventh aspect of the invention is a protein refolding apparatus, wherein the vessels are in the shape of bottles, and a stirring protrusion which protrudes from the inside wall of the vessel along the axial direction is provided inside the vessel.

According to the seventh aspect of the invention, by forming the vessels in the shape of bottles, spillage of the liquid contained therein from an opening can be prevented, and by providing the stirring protrusion on the inside of the vessel, stirring of the liquid inside the vessel can be performed more efficiently and accurately.

An eighth aspect of the invention is a protein refolding apparatus, wherein the stirring protrusion is provided at a predetermined height from the inside bottom of the vessel or higher.

According to the eighth aspect of the invention, the stirring protrusion is provided at a predetermined height from the inside bottom of the vessel or higher. Accordingly, optical measurement can be performed with high accuracy by performing measurement at a position where the protrusion is not present.

A ninth aspect of the invention of the invention is a method of using a protein refolding apparatus comprising: a transport step for transporting either one or two or more vessels capable of holding a protein solution containing a protein to be refolded along a predetermined closed transport path on which the vessels are placed; an optical measurement step for measuring the optical properties of the protein solution within the vessel on the transport path; a pH measuring step for measuring the pH of the protein solution within the vessel on the transport path; and a reagent supply step for supplying a plurality of types of reagents including reagents for adjusting the pH of the protein solution within the vessel, on said transport path, wherein the reagent supply step performs protein refolding by adjusting the pH of the protein solution based on the measurement results of the optical measurement step, the measurement results of the pH measuring step, and a predetermined pH pattern.

According to the ninth aspect of the invention, as is similar to that described for the first aspect of the invention, the protein refolding process is performed non-manually, but without depending completely on a preset processing procedure, by measuring periodically the optical properties and pH value of the protein solution, and advancing the refolding process based on the measurement results. Accordingly, the refolding processes can be performed optimally for a variety of proteins.

According to the present invention, a refolding process can be performed efficiently, cheaply and promptly for large amounts or many types of proteins by simple control, without increasing the burden on the operator.

Furthermore, according to the present invention, by supplying accurate and precise amounts of reagents, calculated based on the measured optical properties and pH value, the refolding process can be advanced with high reliability.

A tenth aspect of the invention is a method of using a protein refolding apparatus, wherein the pH measuring step is executed after the optical measuring step, and the reagent supply step is executed after the pH measuring step.

According to the tenth aspect of the invention, as is similar to that described for the second aspect of the invention, the reagent supply section is provided downstream from the optical measuring section and the pH measuring section in the transport path. Accordingly, the reagent supply section can supply the relevant reagents accurately and precisely based on the measurement results of the optical measurement section and the pH measuring section.

An eleventh aspect of the invention is a method of using a protein refolding apparatus, wherein a stirring step for stirring the contents of the vessels is provided in the reagent supply step, or in the transport path after the reagent supply step.

According to the eleventh aspect of the invention, as is similar to that described for the third aspect of the invention, the reagent supplied to the vessel is thereby mixed with the protein solution, enabling a reaction to be induced in an even and efficient manner.

A twelfth aspect of the invention is a method of using a protein refolding apparatus comprising in the transport path, a suction and transport step for suctioning a predetermined amount of a portion of the liquid contained in the vessel and transporting it to the relevant vessel.

According to the twelfth aspect of the invention, as is similar to that described for the fourth aspect of the invention, it is possible to be aware of the periodic progress of the refolding process, which is useful in the analysis and improvement of the refolding process.

A thirteenth aspect of the invention is a protein refolding apparatus, wherein the reagent supply step can discharge any one of at least an acid reagent, an alkaline reagent and a buffer reagent from at least three reagent discharge nozzles.

According to the thirteenth aspect of the invention, as is similar to that described for the fifth aspect of the invention, accurate pH adjustment is enabled by a simple construction, by the provision of at least three reagent discharge nozzles.

A fourteenth aspect of the invention is a method of using a protein refolding apparatus, wherein in the stirring step, stirring of the liquid in the vessels on the transport path is performed by rotating the vessel about the axis thereof

According to the fourteenth aspect of the invention, as is similar to that described for the sixth aspect of the invention, by stirring the liquid within a vessel by rotating the vessel, stirring can be accomplished by a simple construction and simple control.

A fifteenth aspect of the invention is a method of using a protein refolding apparatus, wherein the stirring step is performed by rotating a vessel in the shape of a bottle on the inside of which is provided a stirring protrusion protruding from the inside wall of the vessel along the axial direction.

According to the fifteenth aspect of the invention, as is similar to that described for the seventh aspect of the invention, by forming the vessels in the shape of bottles, spillage of the liquid contained therein from an opening can be prevented, and by providing the stirring protrusion on the inside of the vessel, stirring of the liquid inside the vessel can be performed more efficiently and accurately.

A sixteenth aspect of the invention is a method of using a protein refolding apparatus, wherein the stirring step involves rotating a vessel in which the stirring protrusion is provided at a predetermined height from the inside bottom of the vessel or higher.

According to the sixteenth aspect of the invention, as is similar to that described for the eighth aspect of the invention, the stirring protrusion is provided at a predetermined height from the inside bottom of the vessel or higher. Accordingly, optical measurement can be performed with high accuracy by performing measurement at a position where the protrusion is not present.

### BRIEF DESCRIPTION OF THE DRAWINGS

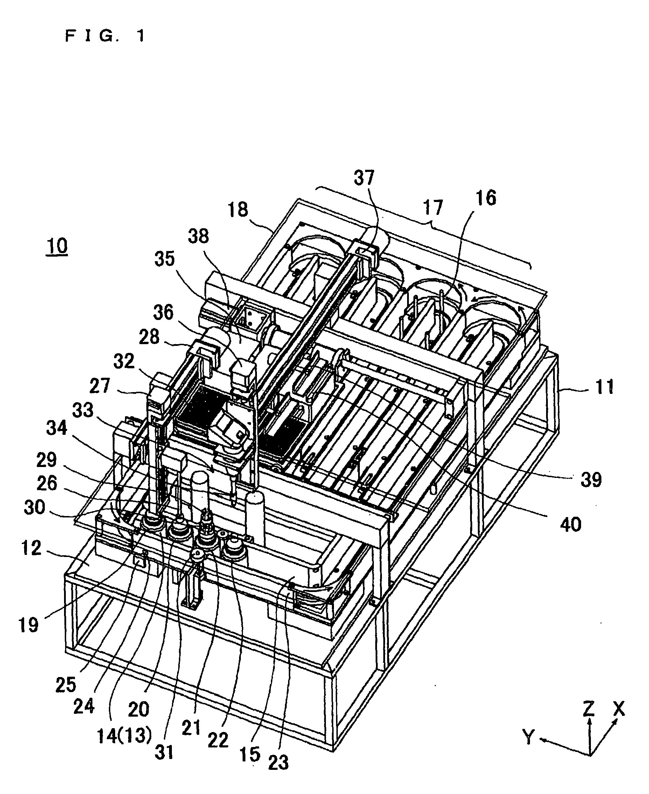

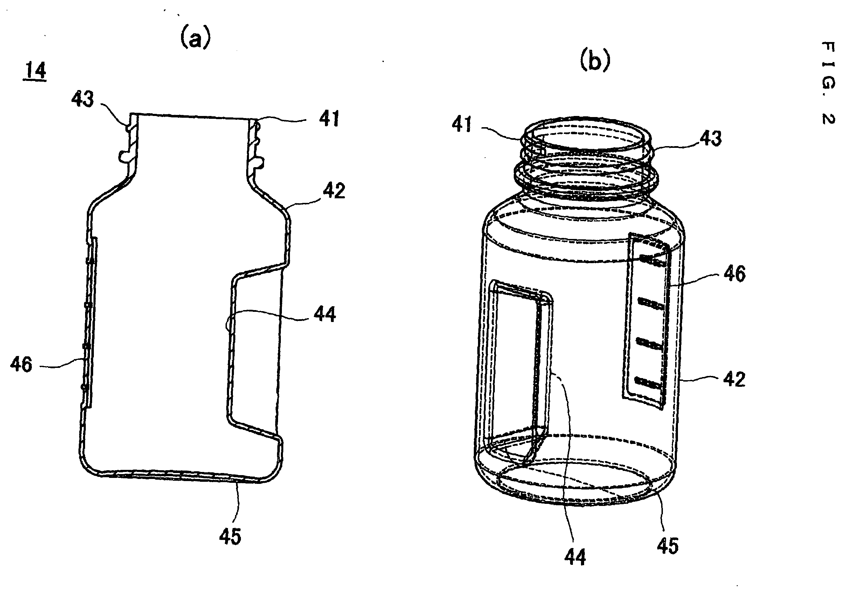

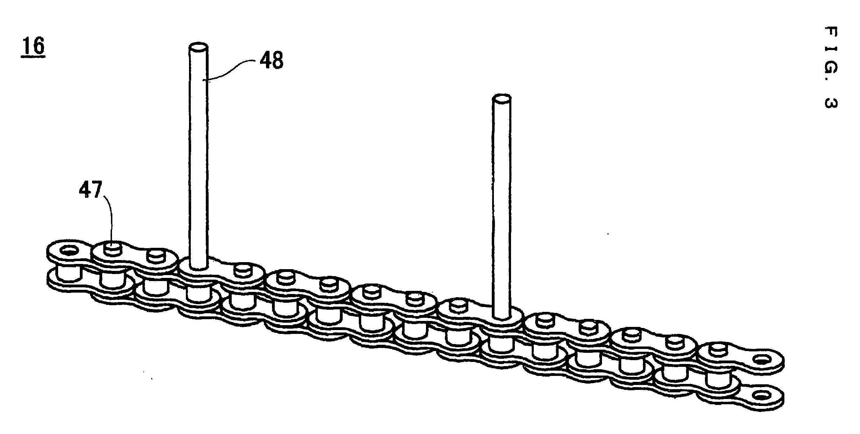

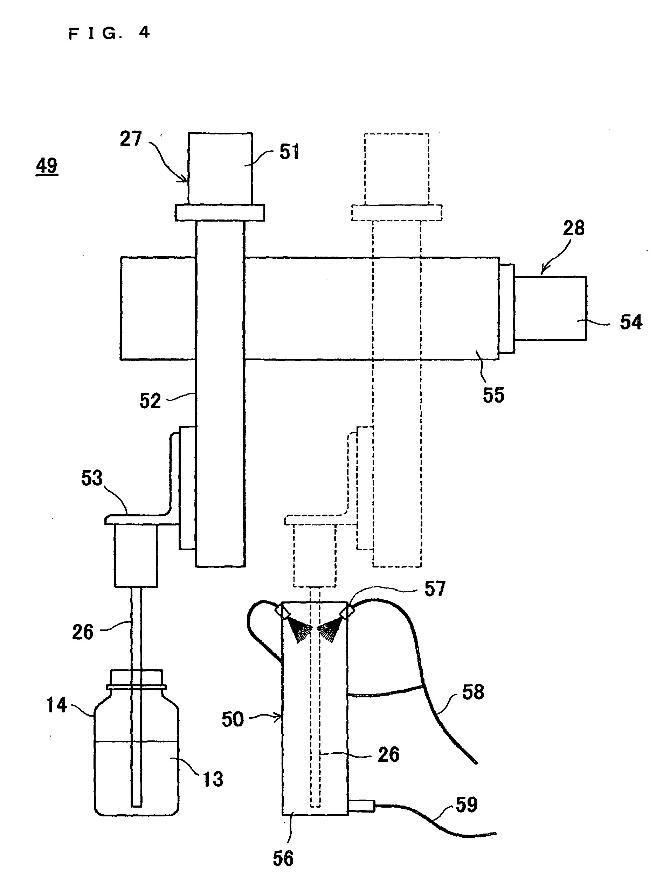

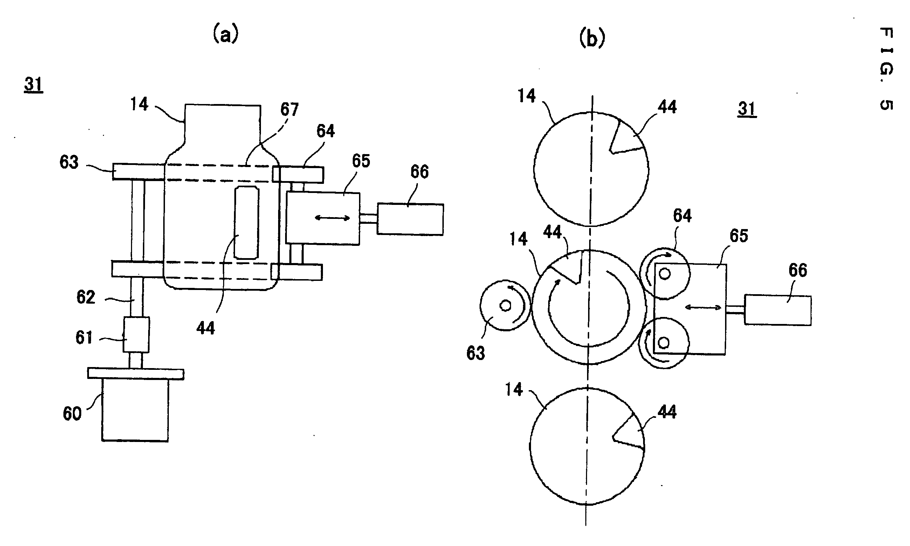

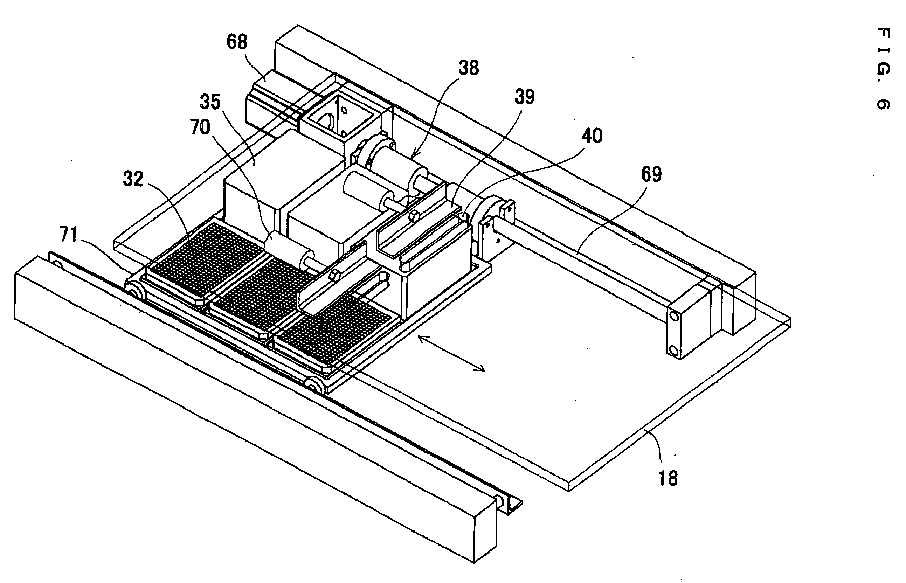

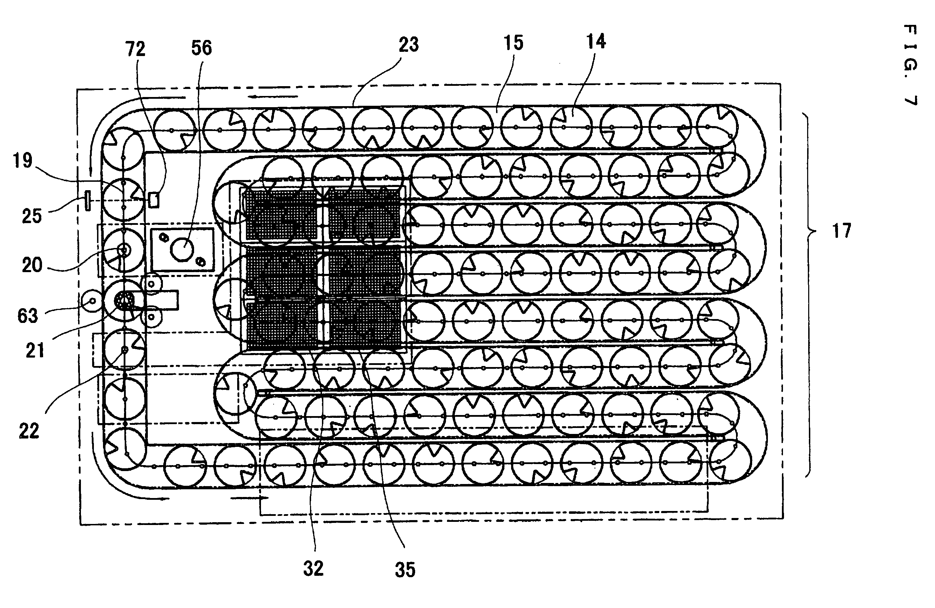

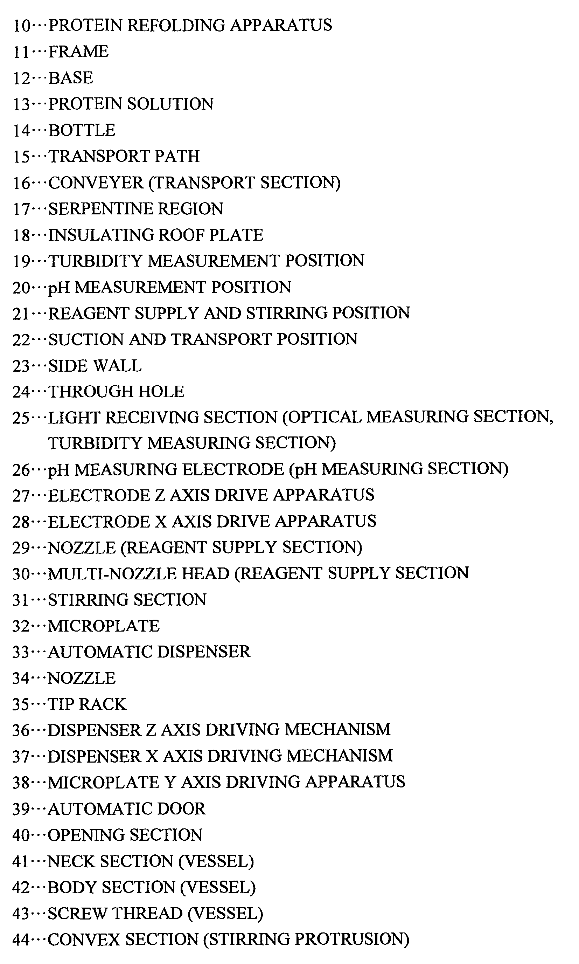

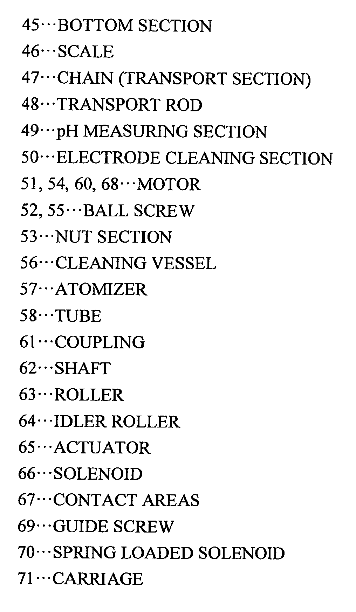

FIG. 1 is a perspective view showing a protein refolding apparatus according to an embodiment of the present invention, FIG. 2 is a diagram showing a bottle according to an embodiment of the present invention, FIG. 3 is a partial perspective view showing a transport apparatus according to an embodiment of the present invention, FIG. 4 is a conceptual diagram showing a pH measuring section according to an embodiment of the present invention, FIG. 5 is a diagram showing a stirring section according to an embodiment of the present invention, FIG. 6 is a partial enlarged perspective view of a protein refolding apparatus according to an embodiment of the present invention, and FIG. 7 is a plan view of a protein refolding apparatus according to an embodiment of the present invention.

### BEST MODE FOR CARRYING OUT THE INVENTION

A protein refolding apparatus 10 according to an embodiment of the present invention is described based on the drawings. This description of the embodiment should not be interpreted as limiting the invention unless particularly specified.

FIG. 1 shows a perspective view of the protein refolding apparatus 10 of the embodiment.

The protein refolding apparatus 10 comprises a frame 11, above which is provided a work base 12 comprising various apparatuses for performing the task of refolding the target protein, and inside the frame 11 beneath the work base 12 are provided various mechanisms not shown in the diagram, including an air conditioning section corresponding to a thermostatic device (a Peltier element or a fan or the like), and a drive circuit for a conveyer acting as the transport section described below.

On the work base 12 are placed either one or a plurality of bottles 14 (a maximum of 96 in this example) corresponding to the vessels, which accommodate a protein solution 13 containing the protein to be processed, and a conveyer (transport section) 16 is provided for transporting the bottles along a predetermined transport path 15. It is preferable that the bottles 14 are made of a transparent material, for example glass, or a resin such as polyethylene, polypropylene or acrylic, so that optical properties such as the turbidity of the protein solution contained therein can be measured. The conveyer 16 transports the bottles 14 together along the transport path 15 in the direction shown by the arrow in the figure.

The transport path 15 forms a closed path, and a part of this closed path includes a serpentine region 17 disposed so as to make the transport path 15 a serpentine path. An insulating roof plate 18 is provided above the entire transport path 15. By setting the temperature of the serpentine region 17 which makes up the majority of the transport path 15, to a predetermined temperature by means of the thermostatic device, the bottles 14 are incubated while they are transported through the serpentine region 17. In the non-serpentine region of the transport path 15, namely those regions excluding the serpentine region 17, in order from the upstream side are provided; a turbidity measurement position 19 where the turbidity of the protein solution 13 contained in the bottles 14 is measured as an optical property, a pH measurement position 20 where the pH of the protein solution 13 contained in the bottles 14 is measured, a reagent supply and stirring position 21 where several predetermined types of reagents can be dispensed for adjusting the pH of the protein solution contained in the bottles 14, and where the protein solution contained in the bottles 14 is stirred, and a suction and transport position 22 where a predetermined amount of the protein solution contained in the bottles 14 is suctioned, transported, and discharged into a corresponding vessel. Holes are provided in the insulating roof plate 18 at the positions 20, 21 and 22 to allow the protein solution 13 in the bottles 14 at these positions to be reached from above.

Two through holes 24 formed in both side walls 23 of the transport path 15 are provided in the turbidity measurement position 19, and an irradiation section (not shown) and a light receiving section 25 are provided on either side of the transport path 15 so that light passes through the through holes 24. Of the light emitted from the irradiation section, the light which passes through the through holes 24 and reaches the light receiving section 25 passes through the lower part of the bottle 14. Turbidity is calculated based on the ratio of the intensity of the light received by the light receiving section 25 to the intensity of the light emitted by the irradiating section.

As described below, a pH measuring electrode 26 is provided at the pH measurement position 20. The pH measuring electrode 26 can be moved in the Z axis direction and the X axis direction in the figure by means of an electrode Z axis drive apparatus 27 and an electrode X axis drive apparatus 28.

A multi-nozzle head 30 with 12 nozzles 29 for supplying various reagents (12 types in this example) necessary to adjust the pH of the protein solution 13 contained in the bottles 14, is provided at the reagent supply and stirring position 21, and each of the nozzles 29 are communicated by tubes with tanks (not shown) provided inside the frame 11, which contain the various reagents.

In addition, a stirring section 31 is provided which stirs the protein solution 13 contained in the bottle 14 positioned at the reagent supply and stirring position 21 by rotating the bottle 14, taking advantage of the difference in angular velocity between the bottle 14 and the protein solution 13 within the bottle 14, and the presence of the protrusion mentioned above.

An automatic dispenser 33 is provided at the suction and transport position 22, which suctions and transports predetermined amounts of the liquid contained in the bottle 14 at the position 22, and discharges the liquid into a predetermined well of a predetermined microplate 32. The automatic dispenser 33 is used with pipette tips (not shown) removably fitted to the nozzle 34 of the automatic dispenser 33. The pipette tips are contained in a tip rack 35 positioned to correspond with the bottles 14, and suction and transport is achieved by exchanging the pipette tips with the relevant pipette tip corresponding to each bottle 14. As a result, cross-contamination between the bottles 14 can be prevented.

The automatic dispenser 33 is driven in the Z axis direction by a dispenser Z axis driving apparatus 36, and driven in the X axis direction by a dispenser X axis driving apparatus 37. As for the Y axis direction, by moving the microplate 32 and the tip rack 35 in the Y axis direction by means of a microplate Y axis driving apparatus 38, the automatic dispenser 33 can be moved relative to the microplate 32 and the like.

In FIG. 1, reference numeral 39 indicates an automatic door which opens and closes an opening 40 provided on the insulating roof plate 18, which is provided so that the nozzle 34 of the automatic dispenser 33, or the pipette tips fitted to the nozzle 34, can reach the pipette tips housed within the tip rack 35, and can reach the wells of the microplate 32.

FIG. 2 shows the bottle 14 in detail.

As shown in the figure, the bottle 14 comprises a neck section 41 and a body section 42, wherein a screw thread 43 which enables a lid (not shown in the figure) to be screwed on, is provided on the neck section 41. A portion of the outside surface of the body section 42 is recessed, and a vertically long convex section 44 of a predetermined length which protrudes inward along the radial direction of the bottle 14 is provided at a predetermined height above a bottom section 45. The convex section 44 corresponds to the stirring protrusion. Moreover, the reason that the convex section 44 is provided at a predetermined height above the bottom section 45 is so that when measuring turbidity, the convex section 44 does not impede the passage of light, and this allows turbidity to be measured accurately.

In addition, a scale 46 is provided on the bottle 14 to display the capacity of the bottle 14.

FIG. 3 shows the main elements of the conveyer 16 in detail. The conveyer 16 comprises a chain 47 laid along the center bottom of the transport path 15, and vertically extending transport rods 48 provided at intervals which allow a bottle 14 to be placed therebetween and transported, for example intervals slightly larger than the diameter of the bottle 14. These transport rods 48 are lower in height than the bottle 14.

FIG. 4 shows a pH measuring apparatus 49 provided at the pH measurement position 20, which measures the pH of the protein solution 13 contained in the bottle 14. As described above, the pH measuring apparatus 49 comprises; the pH measuring electrode 26 which can be inserted into the bottle 14, the electrode Z axis drive apparatus 27 which moves the pH measuring electrode 26 in the Z axis direction, the electrode X axis drive apparatus 28 which moves the pH measuring electrode 26 in the X axis direction, and an electrode cleaning section 50 which cleans the electrode 26 for reuse. The electrode Z axis drive apparatus 28 comprises; a motor 51, a ball screw 52 oriented in the Z axis direction which is held so as to be movable along the X axis direction by the electrode X axis drive apparatus 28, and is rotated by the motor 51, and a nut 53 with the electrode 26 attached, which is screwed onto the ball screw 52 and can be moved vertically by rotation of the ball screw 52.

Furthermore, the electrode X axis drive apparatus 28 comprises a motor 54, and a ball screw 55 oriented in the X axis direction which is rotated by the motor 54. The electrode cleaning section 50 comprises; a cleaning vessel 56 into which the electrode 26 can be inserted along the axial direction, an atomizer 57 in the cleaning vessel 56 which sprays a cleaning solution at the inserted electrode 26, a tube 58 which supplies the cleaning solution to the atomizer 57, and a drainage section 59 which drains the liquid which accumulates in the vessel 56.

With the pH measuring section of the present embodiment, because the electrode is cleaned before measurement, the pH can be measured accurately without requiring manual intervention, and without cross-contamination.

FIG. 5 (a) is a side view of the stirring section 31 provided at the pH stirring position 21, and FIG. 5 (b) is a plan view thereof

The stirring section 31 comprises; a motor 60, a shaft 62 which is rotated by the motor 60 via a coupling 61, a roller 63 which is rotated by the rotation of the shaft 62, the bottle 14 which contacts the side of the roller 63 and is rotated by the roller 63, idler rollers 64 which hold the bottle 14 to prevent it from jittering or shaking, an actuator which moves the idler rollers 64 in the radial direction of the bottle 14 in order to push the idler rollers 64 against the bottle 14, and a solenoid 66 which drives the actuator 65. Reference numeral 67 indicates the areas of the bottle 14 contacted by the rollers.

FIG. 6 is a perspective view showing the protein refolding apparatus 10 according to the present embodiment with a portion removed.

The microplate Y axis driving apparatus 38 comprises a motor 68 and a guide screw 69. Reference numeral 70 in the figure indicates a spring loaded solenoid which opens and closes the automatic door, and reference numeral 71 indicates a carriage driven by the microplate Y axis driving apparatus 38 which transports the microplate 32 and the tip rack 35.

FIG. 7 is an outline plan view of the protein refolding apparatus 10 of the present embodiment.

Although not shown in the figure, this has an information processing apparatus comprising; an arithmetic processing unit with a CPU and memory, a display apparatus, input apparatuses such as a keyboard, switch, mouse and communication device and the like, and output apparatuses such as a printer or memory, for calculating the type and amount of reagent to be supplied by the reagent supply section, based on the measurement results of the optical measurement section, the measurement results of the pH measurement section, and the pH pattern, and for setting of the temperature of the thermostatic device and the speed of the transport section, and for issuing stop and go instructions.

The use of the protein refolding apparatus 10 of the present embodiment will now be described.

Once the maximum amount of the protein has been expressed, it must be separated and purified from the bacterial host. The protein is isolated generally by lysing the cells, for example, by suspending in detergent, adding lysozyme, and then freezing (for example, by suspending cells in 20 ml of TN/1% Triton ™ X-100, adding 10 mg lysozome and freezing at -20°C), thawing and adding DNAase to degrade all of the bacterial DNA, then washing the resulting precipitate in a buffered solution. For refolding, the precipitate is then dissolved in an appropriate solution as discussed below, and placed in a bottle 14 which is then placed on the transport path 15 of the protein refolding apparatus 10.

Here, the pH pattern which the reagent supply section of the protein refolding apparatus 10 should follow, and the reagents which should be supplied from the reagent supply section, are described.

Most published procedures refold proteins using reducing chaotrophs (such as 8 M urea) at a physiological pH, usually pH 7.4 to 8.0. This usually produces large quantities of precipitation or aggregation, making refolding either impossible or achieved with a very low yield. However, it has been found that refolding is possible when the initial refolding pH is high (a substance with a pH higher than 9.0, although a very high pH, such as pH 10, is more desirable). This strategy was initially inspired by the fact that pepsinogen can be reversibly denatured/renatured between pH 8.0 and 9.0.

It is postulated that at a high pH (such as pH 9.0), proteins can obtain some secondary structures, allowing them to be refolded more efficiently when the acidity of the refolding solution is lowered to a biological pH. Later it was found that even for the proteins that could be refolded at a physiological pH, high pH refolding resulted in a better yield. In addition, high pH refolding is an excellent method for preventing initial large scale precipitation. In the present apparatus 10 also, this refolding process in which a high pH is maintained is used as the pH pattern.

Non denaturing concentrations of chaotropic salts such as 0.5 to 1.0 M of urea, guanidine hydrochloride and L-arginine, can be used as the reagents supplied from the reagent supply section. These can be used to assist refolding and activate refolded proteins (Document 2). The preferred concentration of chaotropic reagents is 0.4 M, although the concentration may range from 0 to 4 M. A moderate concentration of urea in the refolding/purification procedures has not been found to have a denaturing effect on proteins.

Furthermore, reagents which can be supplied from the reagent supply section include reducing/oxidizing agents.

Inclusion bodies for mammalian proteins containing disulfide bonds need to be dissolved in the presence of reducing agents. Representative reducing agents include beta-mercaptoethanol, in a range from 0.1 mM to 100 mM, preferably 10 mM; DDT in a range from 0.1 mM to 10 mM, preferably 10 mM; reduced glutathione (GSH) in a range from 0.1 mM to 10 mM, preferably 1 mM; and oxidized glutathione (GSSG), in a range from 0.1 mM to 10 mM, preferably 1 mM. Beta-mercaptoethanol is the preferred reducing agent. In addition, dithiothreitol and/or reduced/oxidized glutathione (GSH, GSSG) can also be included to facilitate "oxido-shuffling" of wrongly folded, intermediate disulfide bonds.

In addition, it is important for the pH pattern that the pH of the protein solution remain at an elevated state long enough for the protein to be refolded. This is preferably achieved by decreasing the pH slowly, in 0.2 pH increments per 24 hours. In this method, the protein solution is adjusted to a high pH, preferably at least 9.0 or higher, to 10 or less preferably 11. The protein is preferably maintained at each pH for at least 24 hours, although comparable effects can be achieved within shorter periods of time, for example two, three, six, nine, twelve, eighteen or twenty hours by adjusting the temperature. The pH is adjusted by adding an acid or the like to the bottle 14 using the multi-nozzle head.

Next, two examples of the refolding process for recombinant proteins are described more specifically.

### (1) Example 1

The first stage is the expression of the recombinant protein.

Expression plasmids should be transfected into an appropriate host, such as the BL21 (DE3) strain of E. coli, and plated on ZB/Ampicillin plates. This selects for the desired recombinant organisms. A single colony from each construct is then inoculated into 100 ml of ZB/ampicillin media and grown for 16 hours at 37°C.

Inoculate 20 ml of the overnight culture into 1 L of LB/ampicillin, and shake at 37°C until the turbidity for light with a wavelength of 600 nm reaches 0.4 to 0.6. Add IPTG to 0.5 mM, and then shake for 3 hours.

Centrifuge, and resuspend cells in 20 ml of TN/1% Triton™ X-100. Add 10 mg lysozome and freeze at -20°C overnight.

Thaw the frozen cells, add 20 µl 1 M MgSO₄, 100 µg DNAase, and stir until the bacterial DNA is completely dissolved.

Add 250 ml of TN/1% Triton, stir from 2 to 4 hours, centrifuge, and repeat the Triton wash one more time. Dissolve the pellet in 10 ml of 8 M urea solution, and add beta-Mercaptoethanol to 100 mM. This solution can be centrifuged, and is then ready for refolding.

Place the resulting solution in the bottle 14, and then place the bottle 14 on the transport path 15 of the protein refolding apparatus 10.

The bottles 14 are transported intermittently on the transport path 15 counterclockwise, and at the turbidity measurement position 19, light with a wavelength of 280 nm is irradiated from the irradiation section 72 onto the lower part of the bottle 14, and received by the light receiving section 25. The turbidity is calculated based on the received light, and whether or not the turbidity for the 8 M urea solution is at the goal of 5.0 is determined.

pH adjustment is repeatedly carried out on the contents of the bottle 14 until the turbidity reaches 5.0. Once the bottle 14 reaches the pH measurement position 20, the electrode 26 is moved by the electrode X axis drive apparatus 28 until positioned directly above the pH measurement position 20. Next, the electrode 26 is lowered into the protein solution 13 inside the bottle 14 by the electrode Z axis drive apparatus 27, and the hydrogen ion concentration in the solution is measured. After measurement is completed, the electrode 26 is lifted, moved in the X axis direction, and then lowered into the cleaning solution vessel 56, where the electrode 26 is cleaned by spraying it with the cleaning solution using the atomizer 57, after which the waste liquid is drained through the drainage section 59. The supply of reagents to adjust the pH is repeated for the bottle 14 until the pH reaches a final value of 10.0.

Once the bottle 14 reaches the reagent supply and stirring position 21, pH adjusting agents are supplied to the bottle 14 via the nozzles 29 of the multi-nozzle head 30 provided at the position 21. For example, predetermined amounts of reagents selected from the following reducing reagents: 10 mM beta-mercaptoethanol, 10 mM DTT (dithiothreitol), 1 mM reduced glutathione (GSH) and 0.1 mH oxidized glutathione (GSSG), are supplied according to the turbidity measurement results and the pH measurement results. While the reagents are being supplied at the reagent supply and stirring position 21, an axial rotation is applied to the bottle 14 at the position 21 by the stirring section 31. In other words, the roller 63 is rotated by a turning force transmitted from the motor 60 via the coupling 61 and the shaft 62, thereby turning the outside surface of the bottle 14, at which time the idler roller 64 is pressed against the bottle 14 by the actuator 65 to prevent it from shaking.

This rotation of the bottle 14 results in effective stirring of the solution inside the bottle 14 by means of the convex section provided inside the bottle 14.

The pH of the solution is adjusted by reagents supplied from the multi-nozzle head until the pH reaches a final value of 10.0.

Next, when the bottle 14 reaches the suction and transport position 22, the nozzle 34 of the automatic dispenser 33 is moved by the dispenser X axis driving mechanism 37 to a position corresponding with the X coordinate of the opening 40, the automatic door 39 of which is open. At the same time, Y axis movement of the carriage 71 positions the nozzle 34, via the opening 40, directly above a position at which an unused pipette tip, which corresponds to the bottle 14, is stored in the tip rack 35. Next, the nozzle 34 is lowered by the dispenser Z axis driving mechanism 36, thereby attaching the pipette tip to the nozzle 34. Then, after the nozzle 34 with the pipette tip attached is raised, the nozzle 34 is moved to the suction and transport position 22 by the dispenser X axis driving mechanism 37, where the pipette tip is lowered into the bottle 14, and a predetermined amount of the protein solution, for example minute amounts of 25 µl at a time, is suctioned, and the nozzle 34 is then once again moved to the X coordinate position corresponding to the opening 40. At the same time, the nozzle 34 is positioned directly above the well in the microplate 32 which corresponds to the bottle 14, via the opening 40, by Y axis movement of the carriage 71. Next the pipette tip is lowered, and the solution therein is discharged into the corresponding well.

After discharging the solution, the nozzle 34 with the pipette tip attached is then positioned at the X axis position corresponding with the opening 40, so that the pipette tip can be stored in an area of the tip rack 35 where the used pipette tips are stored. Y axis movement by the carriage 71 then positions the nozzle 34 directly above the corresponding storage position in the tip rack 35, via the opening 40, and the pipette tip is removed automatically as if pulling off from the nozzle 34.

In this manner, each time the bottle 14 passes the suction and transport position 22 a predetermined amount of the solution is deposited in the wells of the microplate 32. This is used to monitor the flow of the processing of the protein refolding apparatus 10.

After passing the suction and transport position 22, the bottle 14 is transported through the serpentine region 17 of the transport path 15, which make up the majority of the length of the transport path 15, and remains there for a long period of time. The bottle 14 is incubated while passing through the serpentine region 17. The time taken for the bottle 14 to make a complete circuit of the transport path 15 is determined according to the protein to be refolded, and may be two hours or 24 hours, for example.

In this manner, once the protein solution reaches the target turbidity and target pH value, the bottle 14 is again transported along the transport path 15 to the reagent supply and stirring position 21 where 20 mM Tris^{TM} is supplied from one of the nozzles 29, and the resulting solution is rapidly diluted to 20 times the volume of the 20 mM Tris base. In addition, by supplying 1 M HCl from one of the nozzles 29 to obtain a pH of 9.0, the pH is adjusted to a final value of 8.8 within a single circuit, and in the next circuit the pH is adjusted to a value of 8.6, and this adjustment is repeated until a final pH of 8.0 is obtained.

The protein refolded by the apparatus is then concentrated by ultrafiltration, and separated by gel filtration, for example, on a SEPHACRYL™ S-300 column equilibrated with 20 mM TRIS™, HCl 0.4 M urea, pH 8.0. The S-300 fractions can be checked by running a non-reduced SDS-PAGE. The wrongly refolded protein runs at a very high molecular weight, while folded proteins run at a normal molecular weight. The refolded peak from the S-300 column can be further purified with a FPLC Resource-Q™ or Resource-S™ column, which is equilibrated with 20 mM TRIS™-HCl (HEPES buffer for Resource-S™), 0.4 M urea, pH 0.8.

### (2) Example 2

### Expression and Refolding of memapsin 2

Pro-memapsin 2 was PCR amplified and cloned into the *Bam*HI site of a pET11a vector. The resulting vector expresses pro-memapsin 2 having a sequence from Ala-8p to Ala 326. Two expression vectors, pET11-memapsin 2-T1 (hereafter T1) and pET11-memapsin 2-T2 (hereafter T2), were constructed. In both vectors, the N-terminal 15 residues of the expressed recombinant proteins are derived from the expression vector. Pro-memapsin 2 residues start at residue Ala-16. The two recombinant pro-memapsin 2s have different C-terminal lengths. Clone T1 ends at Thr-454 and clone T2 ends at Ala-419. The T1 construct contains a C-terminal extension from the T2 construct but does not express any of the predicted transmembrane domain.

The T1 and T2 expression vectors were separately transfected into E. coli strain BL21 (DE3). The procedures for the culture of transfected bacteria, induction for synthesis of recombinant proteins and the recovery and washing of inclusion bodies containing recombinant proteins are essentially as described by Document 4 (Lin et al., 1994 Methods in Enzymology 214, 195-224). Basically, the inclusion bodies are washed with 1% (v/v) Triton X-100 and 0.15 M NaCl in 0.1 M Tris™ HCl, pH 7.4, the insoluble protein is dissolved in a solution containing 8 M urea, 0.05 M cyclohexylaminopropanesulfonic acid, 10 mM 2-mercaptoethanol, 10 mM DTT (Dithiothreitol), 1 mM reduced glutathione (GSH), 0.1 mM oxidized glutathione (GSSG), 1 mM glycine, and 1 mM ethylenediaminetetraacetic acid (EDTA), pH 10.5, to a protein concentration of about 5 mg/ml. This solution is added dropwise to 20 vol. of rapidly stirred 20 mM Tris base. The pH of the diluted solution is readjusted to 9.0 with 1 M HCl, and kept at 4°C for 24 hr again. This process is repeated until the pH is adjusted to 8.0.

Inclusion bodies are dissolved in 8 M urea, 0.1 M TRIS™, 1 mM Glycine, 1 mM EDTA, 100 mM beta-mercaptoethanol, to pH 10.0.

As described above, the above solution is placed in the bottle 14, which is placed on the transport path 15 of the protein refolding apparatus 10 and transported on the transport path 15 anti-clockwise as shown in FIG. 1. The turbidity for 280 nm wavelengths of the inclusion bodies within the bottle 14 is adjusted to 5.0 using 8 M urea without beta-mercaptoethanol by repeatedly supplying reagents from the nozzles 29 of the multi-nozzle head at the reagent supply and stirring position 21, while measuring the turbidity using the protein refolding apparatus 10 as described above. To obtain the final solution, the following reducing reagents are supplied from the nozzles 29 of the multi-nozzle head 30 at the reagent supply and stirring position 21: 10 mM DTT (dithiothreitol), reduced glutathione with a 1 mM pH, and 0.1 mM oxidized glutathione. The pH of the final solution is 10.0.

The protein solution in the bottle 14 obtained in this manner is rapidly diluted into 20 volumes of 20 mM TRIS™ base, the pH is adjusted to 9.0 by the protein refolding apparatus 10, and the resulting solution is kept at 4°C for 16 hours. The solution is then equilibrated over 6 hours at room temperature, the pH is adjusted to 8.5, and the solution is then returned again to 4°C over 18 hours. The solution is then again equilibrated over 6 hours, the pH adjusted to 8.0, and the solution is then returned again to 4°C over 4 to 7 days. This refolding process is performed using the apparatus 10 described in example 1.

The protein refolded by the apparatus 10 is then concentrated by ultrafiltration, and separated by gel filtration, for example, on a SEPHACRYL™ S-300 column equilibrated with 20 mM TRIS™, HCl 0.4 M urea, pH 8.0. The refolded peak (second peak) from the S-300 column can be further purified with a FPLC Resource-Q™ column, which is equilibrated with 20 mM TRIS™-HCl, 0.4 M urea, pH 0.8. The enzyme is eluted from the column with a linear gradient of NaCl. The refolded peak from the S-300 can also be activated before further purification. For activation, the fractions are mixed with equal volumes of 0.2 M sodium acetate, 70% glycerol, pH 4.0. The mixture is incubated at 22°C for 18 hours, and then dialyzed twice against 20 volumes of 20 mM Bis-TRIS™, 0.4 urea, pH 6.0. The dialyzed materials are then further purified on a FPLC Resource-Q™ column equilibrated with 20 Bis-TRIS™, 0.4 M urea, pH 6.0. The enzyme is eluted with a linear gradient of NaCl.

The embodiments described above are intended to facilitate understanding of the present invention by using specific examples, but these embodiments should not be interpreted as limiting the invention. Accordingly, modifications are possible within a scope which does not alter the gist of the invention. For example, in the description above, only the case in which a specific protein was used is described specifically, but the present invention can be applied to a variety of proteins. Furthermore, above only turbidity was described as an example of an optical property, but other properties which could be measured are the intensity of scattered light, transmittance, reflectance, or spectrum, for example. Furthermore, when describing the thermostatic device a case was described in which the temperature was set to 4°C, but it is of course possible to set this temperature to a different temperature. For example, the refolding process could be accelerated by setting the temperature to 16°C. For example, what took 24 hours above could be done in 2 hours. Moreover, the shape and the like of the constituent elements, the components, and the apparatuses, for example the optical measurement section, the transport section, the vessels, the nozzles of the multi-nozzle head and the nozzles of the dispensing mechanism, are not limited to those shown in the figures.

## Claims

1. A protein refolding apparatus comprising: a transport section on which either one or two or more vessels capable of holding a protein solution containing a protein to be refolded are placed, which transports said vessels along a predetermined closed transport path; an optical measurement section provided on said transport path, which measures the optical properties of the protein solution within said vessel; a pH measuring section provided on said transport path, which measures the pH of the protein solution within said vessel; and a reagent supply section provided on said transport path, capable of supplying each of a plurality of types of reagent including reagents for adjusting the pH of the protein solution within said vessel, wherein
said reagent supply section performs protein refolding by adjusting the pH of said protein solution based on the measurement results of said optical measurement section, the measurement results of said pH measuring section, and a predetermined pH pattern.

2. A protein refolding apparatus according to claim 1, wherein said reagent supply section is provided downstream from said optical measurement section and said pH measuring section.

3. A protein refolding apparatus according to claim 1, wherein a stirring section which stirs the contents of said vessels is provided in said reagent supply section or in the transport path downstream from said reagent supply section.

4. A protein refolding apparatus, wherein a suction and transport section which suctions a predetermined amount of a portion of the protein liquid contained in said vessel and transports it to the relevant vessel, is provided in said transport path.

5. A protein refolding apparatus according to claim 1, wherein said reagent supply section has at least three reagent discharge nozzles, provided so that at least any one of an acid reagent, an alkaline reagent and a buffer reagent can be discharged independently from each reagent discharge nozzle.

6. A protein refolding apparatus according to claim 1, wherein said stirring section stirs the liquid in the vessels on said transport path by rotating said vessel about the axis thereof

7. A protein refolding apparatus according to claim 6, wherein said vessels are in the shape of bottles, and a stirring protrusion which protrudes from the inside wall of said vessel along the axial direction is provided inside said vessel.

8. A protein refolding apparatus according to claim 7, wherein said stirring protrusion is provided at a predetermined height from the inside bottom of said vessel or higher.

9. A method of using a protein refolding apparatus comprising: a transport step for transporting either one or two or more vessels capable of holding a protein solution containing a protein to be refolded along a predetermined closed transport path on which said vessels are placed; an optical measurement step for measuring the optical properties of the protein solution within said vessel on said transport path; a pH measuring step for measuring the pH of the protein solution within said vessel on said transport path; and a reagent supply step for supplying a plurality of types of reagents including reagents for adjusting the pH of the protein solution within said vessel, on said transport path, wherein
said reagent supply step performs protein refolding by adjusting the pH of said protein solution based on the measurement results of said optical measurement step, the measurement results of said pH measuring step, and a predetermined pH pattern.

10. A method of using a protein refolding apparatus according to claim 9, wherein said reagent supply step is performed after said optical measurement step and said pH measuring step.

11. A method of using a protein refolding apparatus according to claim 9, wherein a stirring step for stirring the contents of said vessels is provided in said reagent supply step, or in the transport path after said reagent supply step.

12. A method of using a protein refolding apparatus according to claim 9, comprising in said transport path, a suction and transport step for suctioning a predetermined amount of a portion of the liquid contained in said vessel and transporting it to the relevant vessel.

13. A protein refolding apparatus according to claim 9, wherein said reagent supply step can discharge independently any one of at least an acid reagent, an alkaline reagent and a buffer reagent from at least three reagent discharge nozzles.

14. A method of using a protein refolding apparatus according to claim 9, wherein in said stirring step, stirring of the liquid in the vessels on said transport path is performed by rotating said vessel about the axis thereof.

15. A method of using a protein refolding apparatus according to claim 9, wherein said stirring step is performed by rotating a vessel in the shape of a bottle on the inside of which is provided a stirring protrusion protruding from the inside wall of the vessel along the axial direction.

16. A method of using a protein refolding apparatus according to claim 9, wherein said stirring step involves rotating a vessel in which said stirring protrusion is provided at a predetermined height from the inside bottom of said vessel or higher.
